# EUROPEAN PATENT APPLICATION

(11) **EP 4 046 642 A1**
(43) Date of publication of application: **24.08.2022**
(21) Application number: 20877712.8
(22) Date of filing: 16.10.2020
(51) Int. Cl.: A61K 35/34, A61L 27/36, A61L 27/38, A61P 1/04, A61P 29/00, C12N 5/077

(54) **CELL CULTURE FOR TREATING INFLAMMATORY DISEASE**

(30) Priority: 17.10.2019 JP 2019190479
(71) Applicant: TERUMO Kabushiki Kaisha, Tokyo 151-0072 (JP)
(72) Inventor: OHASHI, Fumiya, Ashigarakami-gun, Kanagawa 259-0151 (JP); MATSUMURA, Masaki, Ashigarakami-gun, Kanagawa 259-0151 (JP)
(74) Representative: Prüfer & Partner mbB Patentanwälte · Rechtsanwälte
(86) International application number: PCT/JP2020/039014
(87) International publication number: WO 2021/075525

(57) **Abstract**

An object of the present invention is to provide a cell culture, a method for producing the cell culture, a kit for producing the cell culture, a method for treating inflammatory disease using the cell culture, and the like, for treating inflammatory disease. A cell culture containing cells derived from skeletal muscle, a method for producing the cell culture containing cells derived from skeletal muscle, a kit for producing the cell culture containing cells derived from skeletal muscle, a method for treating inflammatory disease using the cell culture, and the like solve the above problem.

## Description

### Technical Field

The present invention relates to a cell culture containing cells derived from skeletal muscle, a method for producing the cell culture, a kit for producing the cell culture, and a method for treating inflammatory disease using the cell culture, and the like, for treating inflammatory disease.

### Background Art

In recent years, to repair damaged tissues or the like, attempts to transplant various cells have been made. For example, for repairing myocardial tissues damaged due to an ischemic heart disease such as angina pectoris or myocardial infarction, attempts have been made to utilize fetal cardiomyocytes, myoblast cells, mesenchymal stem cells, cardiac stem cells, embryonic stem (ES) cells, induced pluripotent stem (iPS) cells, and the like (Non Patent Literature 1).

As an example of such attempts, sheet-shaped cell cultures, which are obtained by forming cells into a sheet shape, have been developed, and some of the sheet-shaped cell cultures are in the stage of clinical application. Examples of such clinical application include a sheet-shaped cell culture, which is used by attaching to the outside of a treatment site, in order to prevent perforation or the like after endoscopic submucosal dissection (Patent Literature 1), and a sheet-shaped cell culture containing adipocytes, which improves cardiac function in a patient with heart disease by adiponectin secreted (Patent Literature 2).

Inflammatory bowel disease is an intractable disease of which the cause of the onset has not been clearly understood, and includes mainly Crohn disease, and ulcerative colitis. At present, it is estimated that there are more than 220,000 patients in total with Crohn disease or ulcerative colitis in Japan. As the method for treating such a disease, at present, for example, administration of a steroid drug, an immunosuppressive agent, an anti-TNF-a antibody preparation, or the like has been performed.

As the new therapeutic method for inflammatory bowel disease, a method of administering mesenchymal stem cells having various actions such as tissue differentiation, angiogenesis, and regulation of immune function has been proposed. For example, a method of administering intravenously or intralymphatically mesenchymal stem cells or adipose stem cells to an animal model of enteritis or chronic rheumatoid arthritis (Patent Literatures 3 to 5), a method of administering intestinally or intravenously a culture supernatant of mesenchymal stem cells to a rat model of enteritis (Patent Literatures 6 and 7), a method of regulating peripheral immune response by mesenchymal stem cells into which genes have been transferred (Patent Literature 8), and the like are known.

### Citation List

### Patent Document

Patent Document 1: Japanese Patent Application No. 2018-140977
Patent Document 2: JP 5661048 B
Patent Document 3: JP 2017-35094 A
Patent Document 4: Japanese Patent Application No. 2009-7321
Patent Document 5: P 6512759 B
Patent Document 6: JP 2017-137268 A
Patent Document 7: JP 6132459 B
Patent Document 8: JP 2019-6790 A

### Non Patent Document

Non Patent Document 1: Haraguchi et al., Stem Cells Trans1 Med. 2012 Feb; 1(2): 136-41

### Summary of Invention

### Technical Problem

An object of the present invention is to provide a cell culture containing cells derived from skeletal muscle, a method for producing the cell culture, a kit for producing the cell culture, a method for treating inflammatory disease using the cell culture, and the like, for treating inflammatory disease.

### Solution to Problem

In the treatment of inflammatory bowel disease using mesenchymal stem cells, there remains a problem that it is difficult to culture mesenchymal stem cells suitable for a subject to be administered in an amount that can be administered as a drug, the bioadhesiveness at a desired site is insufficient when mesenchymal stem cells are administered intravenously or intralymphatically, and the like.

The present inventors have found that a culture supernatant of a sheet containing cells derived from skeletal muscle contains a component useful for treatment of inflammatory bowel disease, such as myokine, and as a result of further studies based on such a finding, the present inventors have completed the present invention.

That is, the present invention relates to the following:
[1] A cell culture for treating inflammatory disease, including cells derived from skeletal muscle.
[2] The cell culture described in [1], in which the cell culture is a sheet-shaped cell culture.
[3] The cell culture described in [1] or [2], in which the cell culture secretes myokine.
[4] The cell culture described in any one of [1] to [3], in which the inflammatory disease is generated in the lower gastrointestinal tract.
[5] The cell culture described in any one of [1] to [4], in which the inflammatory disease is inflammatory bowel disease.
[6] The cell culture described in any one of [1] to [5], in which the cell culture is used for transplantation to the serosal side of the gastrointestinal tract.
[7] A method for producing the sheet-shaped cell culture described in any one of [2] to [6], including:
   seeding cells on a substrate;
   forming the seeded cells into a sheet-shaped cell culture; and
   detaching the formed sheet-shaped cell culture from the substrate.
[8] A kit for producing the cell culture described in any one of [1] to [6], including: cells; and a culture medium and a substrate, for culturing the cells.
[9] A method for treating inflammatory disease, including transplanting a cell culture containing cells derived from skeletal muscle.
[10] The method described in [9], in which the cell culture is a sheet-shaped cell culture.
[11] The method described in [9] or [10], in which the cell culture secretes myokine.
[12] The method described in any one of [9] to [11], in which the inflammatory disease is generated in the lower gastrointestinal tract.
[13] The method described in any one of [9] to [12], in which the inflammatory disease is inflammatory bowel disease.
[14] The method described in any one of [9] to [13], in which transplantation of the cell culture is transplantation to the serosal side of the gastrointestinal tract.

### Advantageous Effects of Invention

According to the present invention, by performing transplantation of a cell culture containing cells derived from skeletal muscle, the cure of inflammatory disease can be promoted.

### Brief Description of Drawings

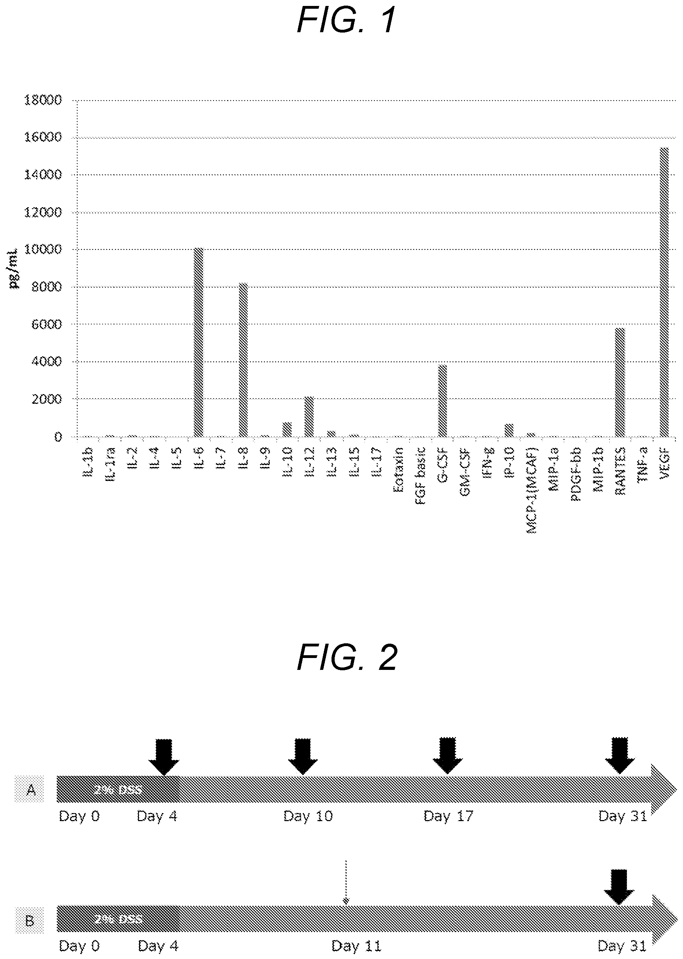

Fig. 1 shows amounts of various cytokines contained in a culture supernatant of a sheet-shaped cell culture containing human myoblast cells, which are measured by using Bio-Plex (trademark) multisystem.
Fig. 2 shows a plan for a transplant test in a mouse model of colitis. Group A shows a follow-up group, and group B shows a test group. 2% dextran sulfate sodium (DSS) is administered in free drinking water in both of the groups from the start date of the test to the 4th day. The thin arrow in the diagram indicates the day when a sheet-shaped cell culture was transplanted, and the thick arrows indicate the days when the autopsy was performed.

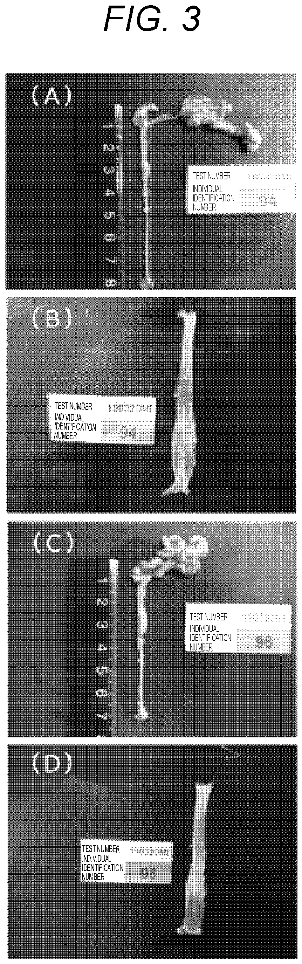

Fig. 3 shows photographs of tissues collected from mice in a sheet-shaped cell culture transplantation group and in a sham surgery group by autopsy on the 31st day after the start of the test. (A) is a photograph of the lower gastrointestinal tract taken out from a mouse in the sheet-shaped cell culture transplantation group, and (B) is a photograph of the inner membrane of the gastrointestinal tract confirmed by cutting open the lower gastrointestinal tract. (C) is a photograph of the lower gastrointestinal tract taken out from a mouse in the sham surgery group, and (D) is a photograph of the inner membrane of the gastrointestinal tract confirmed by cutting open the lower gastrointestinal tract.

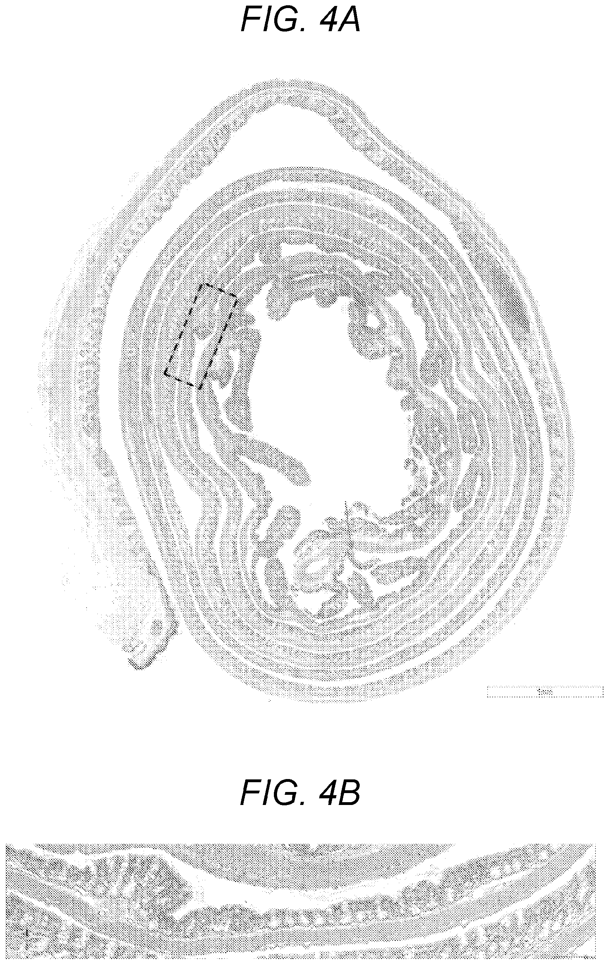

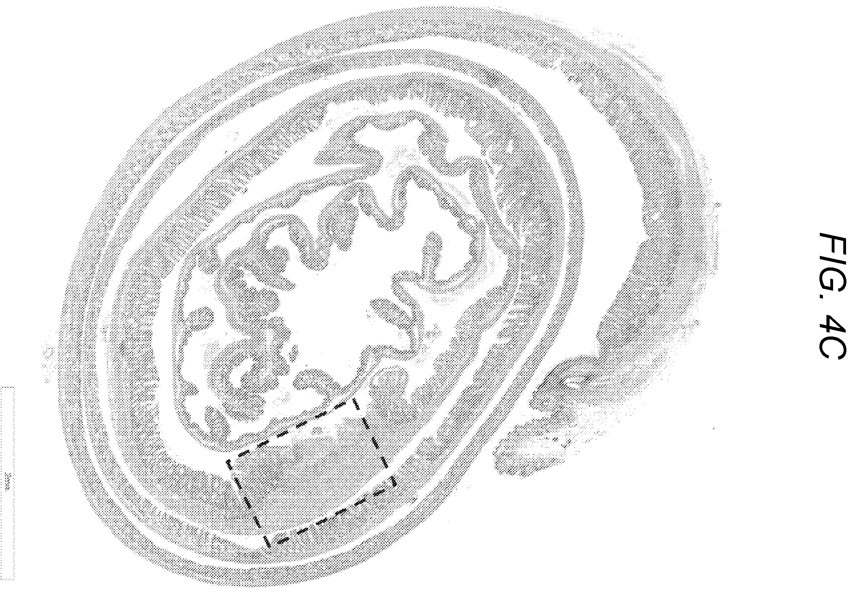

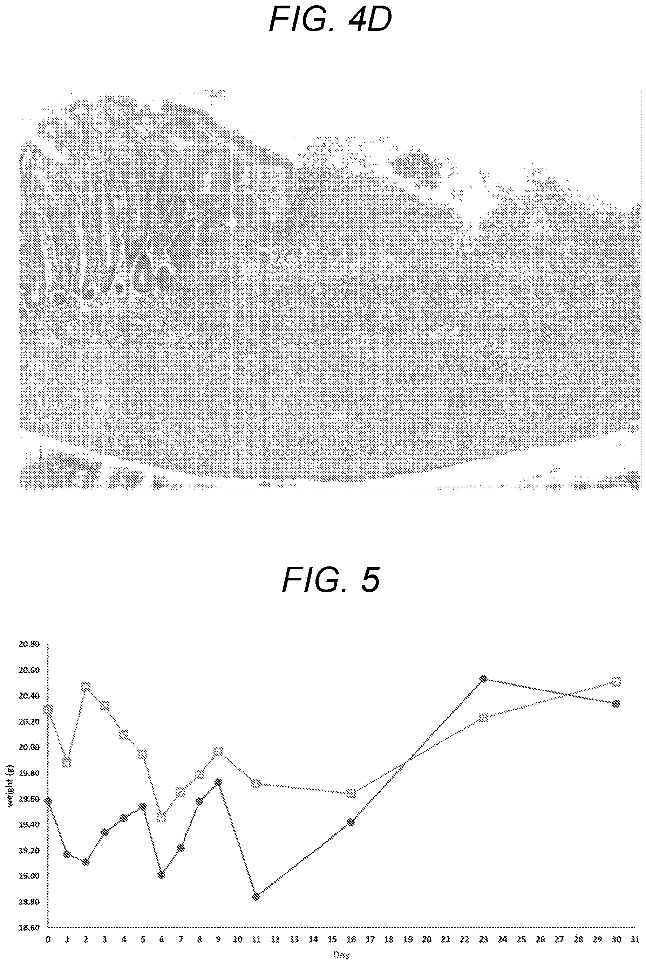

Fig. 4 shows images of colon tissues of mice in the sheet-shaped cell culture transplantation group and in the sham surgery group, obtained by autopsy on the 31st day after the start of the test. Fig. 4A shows an image of a cross section of a mouse in the sheet-shaped cell culture transplantation group obtained by autopsy on the 31st day after the start of the test.
Fig. 4B is an enlarged image of a portion surrounded by a dashed line in Fig. 4A.
Fig. 4C shows an image of a cross section of a mouse in the sham surgery group obtained by autopsy on the 31st day after the start of the test.
Fig. 4D is an enlarged image of a portion surrounded by a dashed line in Fig. 4C.
Fig. 5 is a graph showing changes in the body weight of the sheet-shaped cell culture transplantation group and the sham surgery group. The squares indicate the sham surgery group, and the black circles indicate the sheet-shaped cell culture transplantation group. The vertical axis shows the body weight of a mouse, and the horizontal axis shows the number of days elapsed after the start of the test. On the 11th day after the start of the test, transplantation of a sheet-shaped cell culture was performed.

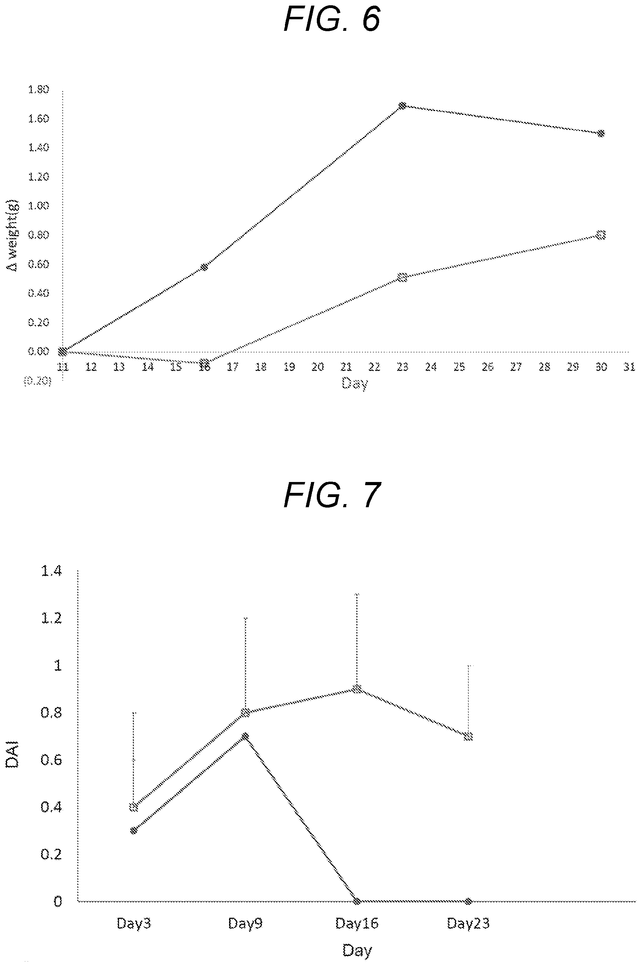

Fig. 6 is a graph showing changes in the body weight of the sheet-shaped cell culture transplantation group and the sham surgery group, using the body weight of each mouse on the 11th day after the start of the test as the basis. The squares indicate the sham surgery group, and the black circles indicate the sheet-shaped cell culture transplantation group. The vertical axis shows the changes in the body weight from the basis, and the horizontal axis shows the number of days elapsed after the start of the test.
Fig. 7 is a graph showing inflammation scores of mice in the sheet-shaped cell culture transplantation group and in the sham surgery group. The vertical axis shows the value of inflammation score (DAI), and the horizontal item axis shows the date when the score was measured. The squares indicate the sham surgery group, and the black circles indicate the sheet-shaped cell culture transplantation group.

### Description of Embodiments

Hereinafter, the present invention will be described in detail.

One aspect of the present invention relates to a cell culture containing cells derived from skeletal muscle, for treating inflammatory disease.

In the present invention, the inflammatory disease is a disease characterized by symptoms of inflammation. Examples of the inflammatory disease include, but are not limited to, a systemic or local inflammation disease such as an allergy, or an immune complex disease, a gastrointestinal disease such as Crohn disease, ulcerative, acute or ischemic colitis, intestinal Behcet disease, diverticulitis, or cholecystitis, a skin-related disease such as dermatitis, a cardiovascular disease such as endocarditis, or myocarditis, a respiratory disease such as asthma, tuberculosis (TB), bronchiectasis, or chronic obstructive pulmonary disease (COPD), a connective tissuerelated disease such as rheumatoid arthritis, osteomyelitis, or fasciitis, a urogenital system disease such as nephritis, a nervous system-related disease such as meningitis, encephalitis, or multiple sclerosis, a disease caused by infection with viruses, fungi, or microorganisms, an autoimmune disease such as thyroiditis, and a cancer.

In one embodiment, the inflammatory disease particularly refers to a disease resulting from abnormal release of inflammatory cytokines. Preferably, the inflammatory disease in the present invention refers to inflammatory bowel disease.

In the present invention, the expression "cell culture" refers to a composition obtained through a cell culture step. In one embodiment, the cell culture of the present invention is a sheet-shaped cell culture. In the present invention, the expression "sheet-shaped cell culture" means a cell culture in a sheet shape formed by connecting cells to each other. The cells may be connected to each other directly (including connection through a cellular element such as an adhesion molecule) and/or through a mediator (interposed materials). The mediator is not particularly limited as long as it is a substance that can at least physically (mechanically) connect cells to each other, and as the mediator, for example, an extracellular matrix or the like can be mentioned. The mediator is preferably derived from a cell, and particularly derived from a cell that forms a cell culture. The cells are at least physically (mechanically) connected to each other, but may be more functionally, for example, chemically electrically connected to each other. The sheet-shaped cell culture may be formed of one cell layer (single layer), or may also be formed of two or more cell layers (such as a laminated (multilayer) body, for example, two-layer, three-layer, four-layer, five-layer, or six-layer). Further, the sheet-shaped cell culture may have a three-dimensional structure having a thickness exceeding the thickness of one cell without showing any clear layered structure of the cells. For example, in the vertical section of the sheet-shaped cell culture, the cells may be present in a state of being non-uniformly (for example, mosaic-like) arranged without being uniformly aligned in the horizontal direction.

The sheet-shaped cell culture preferably does not contain a scaffold (support). The scaffold may be used in some cases in the technical field to which the invention belongs in order to attach cells onto the surface of and/or to the inside of the scaffold and to maintain the physical integrity of the sheet-shaped cell culture, and as the scaffold, for example, a membrane made of polyvinylidene difluoride (PVDF) or the like is known, but the sheet-shaped cell culture of the present disclosure can maintain the physical integrity even without such a scaffold. Further, the sheet-shaped cell culture of the present disclosure preferably consists only of cell-derived substances forming the sheet-shaped cell culture, and does not contain any other substances.

In the present invention, the cell culture contains cells derived from skeletal muscle. In the present invention, the cells derived from skeletal muscle refer to satellite cells, myoblast cells, skeletal muscle cells, skeletal myotubes, and skeletal muscle fibers. Preferably, the cells derived from skeletal muscle are myoblast cells. The cells forming the cell culture of the present invention contain cells derived from skeletal muscle by 50% or more, 60% or more, 65% or more, 70% or more, 75% or more, 80% or more, 85% or more, or 90% or more, and preferably 60% or more.

The myoblast cells are well known in the technical field to which the present invention belongs, and can be prepared from skeletal muscle by any known method (for example, method disclosed in JP 2007-89442 A, or the like), and as the myoblast cells, for example, catalog number: CC-2580 of Lonza Japan, product code 3520 of Cosmo Bio Co., Ltd., or the like can be obtained commercially. The myoblast cells are not limited to such cells, and can be identified by a marker such as CD56, α7 integrin, myosin heavy chain IIa, myosin heavy chain IIb, myosin heavy chain IId (IIx), MyoD, Myf5, Myf6, myogenin, desmin, or PAX3. In one embodiment, the myoblast cells are CD56 positive. In one embodiment, the myoblast cells are CD56 positive and desmin positive.

In a case where the myoblast cells are prepared from striated muscle tissue, the prepared cell population contains fibroblast cells. When the cell culture according to the present invention is produced, in a case where a cell population containing the myoblast cells prepared from striated muscle tissue is used, a certain amount of fibroblast cells is contained in the cell population. The fibroblast cells are well known in the technical field to which the present invention belongs, and can be identified by a marker such as TE-7 (see, for example, Rosendaal et al., J Cell Sci. 1994, 107(Pt1): 29-37, Goodpaster et al. J Histochem Cytochem. 2008, 56(4): 347-358, and the like).

In one embodiment, the cells forming the cell culture of the present invention include the myoblast cells prepared from striated muscle tissue. Accordingly, the cell population used in production of the cell culture of the present invention can contain myoblast cells and fibroblast cells. In one embodiment, the cell population used in production of the cell culture of the present invention can have a CD56-positive rate of 50% or more, 60% or more, 65% or more, 70% or more, 75% or more, 80% or more, 85% or more, or 90% or more, and preferably 60% or more.

The cell population used in production of the cell culture of the present invention can contain fibroblast cells, and in a case where the content of the fibroblast cells is extremely high, the content of myoblast cells is lowered, and thus, this is not preferable. Accordingly, in one embodiment, the cell population used in production of the cell culture of the present invention can have a TE-7 positive rate of 50% or less, 40% or less, 35% or less, 30% or less, 25% or less, 20% or less, 15% or less, or 10% or less, and preferably 40% or less.

The cell population used in production of the cell culture of the present invention can contain cells other than the myoblast cells and the fibroblast cells, but the smaller the number of such cells is, the more preferable the cell population is. Accordingly, the higher the total value of the CD56-positive rate and the TE-7 positive rate is, the more preferable the cell population is, and the total value can be, for example, 80% or more, 85% or more, 90% or more, 95% or more, 96% or more, 97% or more, 98% or more, or 99% or more, and preferably 90% or more.

In the present invention, a cytokine is secreted from a cell culture containing cells derived from skeletal muscle.

In the present invention, the cytokine secreted from a cell culture containing cells derived from skeletal muscle is commonly referred to as a myokine, and examples of such a cytokine include, but are not limited to, IL-1b, IL-1ra, IL-2, IL-4, IL-5, IL-6, IL-7, IL-8, IL-9, IL-10, IL-12, IL-13, IL-15, IL-17, eotaxin, FGF-basic, G-CSF, GM-CSF, IFN-g, IP-10, MCP-1 (MCAF), MIP-1a, PDGF-bb, MIP-1b, RANTES, TNF-a, HGF-1, SDF-1, and VEGF.

In a case where the cell culture of the present invention is a sheet-shaped cell culture, the thickness of the sheet-shaped cell culture is not particularly limited. In a case where a single-layer sheet is used as the sheet-shaped cell culture, the thickness is usually a thickness of one or more cells, and varies depending on the kind of the cells forming the sheet-shaped cell culture, and in one embodiment, the sheet-shaped cell culture of the present invention has a thickness of 30 µm or more, and in one preferred embodiment, the sheet-shaped cell culture has a thickness of 50 µm or more. Examples of the range of the value of the sheet-shaped cell culture of the present invention include 30 µm to 200 µm, preferably 50 µm to 150 µm, and more preferably 60 µm to 100 µm. In a case where a laminated sheet is used as the sheet-shaped cell culture, the thickness does not exceed the value obtained by a thickness of the single-layer sheet × the number of laminated sheets. Accordingly, as one embodiment, in a case where, for example, a sheet obtained by stacking five single-layer sheets in layers is used, the sheet has a thickness of 150 µm or more, and in one preferred embodiment, the sheet has a thickness of 250 µm or more. In that case, examples of the range of the value of the sheet-shaped cell culture of the present invention include 150 µm to 1000 µm, preferably 250 µm to 750 µm, and more preferably 300 µm to 500 µm.

The cells composing the cell culture of the present invention can be derived from any organism that is to be subject to treatment with the cell culture, and examples of such an organism include, but are not limited to, a human, primates, a dog, a cat, a pig, a horse, a goat, a sheep, rodent animals (such as a mouse, a rat, a hamster, and a guinea pig), and a rabbit. Further, as the cells composing the cell culture of the present invention, only one kind of, or two or more kinds of cells may be used. In a preferred embodiment of the present invention, in a case where there are two or more kinds of the cells that form the cell culture, the content ratio (purity) of the most abundant cells is 60% or more, preferably 70% or more, and more preferably 75% or more, at the end of the cell culture production.

The cells may be cells derived from heterogeneous cells, or may also be cells derived from homogeneous cells. In this regard, in a case where the cell culture is used for transplantation, the expression "cells derived from heterogeneous cells" means cells derived from an organism of a species different from that of the recipient. For example, in a case where the recipient is a human, cells derived from a monkey or a pig correspond to the cells derived from heterogeneous cells. Further, the expression "cells derived from homogeneous cells" means cells derived from an organism of the same species as that of the recipient. For example, in a case where the recipient is a human, human cells correspond to the cells derived from homogeneous cells. The cells derived from homogeneous cells include self-derived cells (also referred to as "self cells" or "autologous cells"), that is, recipient-derived cells, and cells derived from homogeneous non-self cells (also referred to as "non-autologous cells"). The self-derived cells are preferable in the present disclosure because the cells do not cause any rejection even when being transplanted. However, cells derived from heterogeneous cells, or cells derived from homogeneous non-self cells can also be used. In a case where such cells derived from heterogeneous cells or derived from homogeneous non-self cells are used, the cells may require immunosuppressive treatment to suppress the rejection, in some cases. Note that in the present specification, the cells other than the self-derived cells, that is, cells derived from heterogeneous cells and cells derived from homogeneous non-self cells may also be collectively referred to as "non-self-derived cells". In one embodiment of the present disclosure, the cells are autologous cells or non-autologous cells. In one embodiment of the present disclosure, the cells are autologous cells. In another embodiment of the present disclosure, the cells are non-autologous cells.

In the present invention, in a case where the cell culture is a sheet-shaped cell culture, the sheet-shaped cell culture can be produced by any method known to a person skilled in the art (see, for example, Patent Literature 1, JP 2010-081829 A, JP 2011-110368 A, and the like). The method for producing a sheet-shaped cell culture typically includes, but is not limited to, a step of seeding cells on a substrate, a step of forming the seeded cells into a sheet-shaped cell culture, and a step of detaching the formed sheet-shaped cell culture from the substrate. Before the step of seeding cells on a substrate, a step of freezing cells and a step of thawing the cells may be performed. Further, a step of washing the cells may be performed after the step of thawing the cells. Each of these steps can be performed by any known technique suitable for the production of a sheet-shaped cell culture. The production method of the present disclosure may include a step of producing a sheet-shaped cell culture, and in that case, the step of producing a sheet-shaped cell culture may include 1 or 2 or more of the steps according to the method for producing a sheet-shaped cell culture as sub-steps. In one embodiment, a step of proliferating the cells is not included after the step of thawing the cells and before the step of seeding the cells on a substrate.

In one embodiment, in the cell culture of the present invention, cells are connected to each other through an extracellular matrix produced by the cells forming the cell culture. In one embodiment, the cell culture of the present invention contains an extracellular matrix. In the present invention, although depending on various conditions, the extracellular matrix produced by the cells forming a cell culture is generated, for example, by culturing the cells for 24 hours or more, for example, for 24 hours, 30 hours, 36 hours, 42 hours, 48 hours, 54 hours, 60 hours, 66 hours, or 72 hours, after seeding the cells.

The extracellular matrix contributes to the binding of a cell culture to a transplant site, and can simplify the step of binding the cell culture to a transplant site, such as suturing, when the cell culture is transplanted. Further, the extracellular matrix is a cell-derived component, and thus, there is no problem in use of the extracellular matrix for transplantation together with the cell culture according to the present invention.

Examples of the substrate are not particularly limited as long as cells can form a cell culture on the substrate, and include containers made of various materials, and a solid or half-solid surface in a container. It is preferable that the container has a structure/material that does not allow a liquid such as a liquid culture medium to permeate. Examples of the material include, but are not limited to, polyethylene, polypropylene, Teflon (registered trademark), polyethylene terephthalate, polymethyl methacrylate, nylon-6,6, polyvinyl alcohol, cellulose, silicon, polystyrene, glass, polyacrylamide, polydimethyl acrylamide, and a metal (for example, iron, stainless steel, aluminum, copper, or brass). Further, it is preferable that the container has at least one flat surface. As such a container, without any limitation, for example, a culture container provided with the bottom made of a substrate capable of forming a cell culture and the liquid-impermeable side can be mentioned. Specific examples of the culture container include, but are not limited to, a cell-culture dish, and a cell-culture bottle. The bottom of the container may be transparent or opaque. If the bottom of a container is transparent, cells can be observed and counted from the underside of the container. Further, the container may have a solid or half-solid surface inside thereof. Examples of the solid surface include a plate, and a container, which are made of various materials as described above, and examples of the half-solid surface include a gel, and a soft polymer matrix. As the substrate, a substrate prepared by using the above-described materials may be used, or a commercially available material may be used. As the preferred substrate, without any limitation, for example, a substrate having an adhesive surface, which is suitable for forming a sheet-shaped cell culture, can be mentioned. Specifically, a substrate having a hydrophilic surface, for example, a substrate of which the surface is coated with a hydrophilic compound such as corona discharge-treated polystyrene, a collagen gel, or a hydrophilic polymer, a substrate of which the surface is coated with an extracellular matrix of collagen, fibronectin, laminin, vitronectin, proteoglycan, glycosaminoglycan, or the like, or a cell adhesion factor such as a cadherin family, a selectin family, or an integrin family, or the like can be mentioned. Further, such a substrate is commercially available (for example, Corning (registered trademark) TC-Treated Culture Dish, Corning, or the like). The overall or part of the substrate may be transparent or opaque.

The surface of the substrate may be coated with a material of which the physical properties change in response to a stimulus, for example, temperature or light. As such a material, without any limitation, a known material, for example, a temperature-responsive material made of a homopolymer or a copolymer of a (meth)acrylamide compound, a N-alkyl-substituted (meth)acrylamide derivative (for example, N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-ethoxyethyl acrylamide, N-ethoxyethyl methacrylamide, N-tetrahydrofurfuryl acrylamide, N-tetrahydrofurfuryl methacrylamide, or the like), a N,N-dialkyl-substituted (meth)acrylamide derivative (for example, N,N-dimethyl (meth)acrylamide, N,N-ethyl methyl acrylamide, N,N-diethyl acrylamide, or the like), a (meth)acrylamide derivative having a cyclic group (for example, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, 4-(1-oxo-2-methyl-2-propenyl)-morpholine, or the like), or a vinyl ether derivative (for example, methyl vinyl ether), or a photoresponsive material such as a light-absorbing polymer having an azobenzene group, a copolymer of a vinyl derivative of triphenylmethane leucohydroxide and an acrylamide-based monomer, or N-isopropylacrylamide gel containing spirobenzopyran, may be used (see, for example, JP H02-211865 A, and JP 2003-33177 A). By giving a predetermined stimulus to such a material, the physical properties, for example, the hydrophilicity and the hydrophobicity can be changed, and the detachment of a cell culture attached on the material can be promoted. A culture dish coated with a temperature-responsive material is commercially available (for example, UpCell (registered trademark) of CellSeed Inc., or Cepallet (registered trademark) of DIC Corporation), and such a culture dish can be used for the production method of the present disclosure.

The substrate may have various shapes, but is preferably flat. Further, the area is not particularly limited, and may be, for example, around 1 cm² to around 200 cm², around 2 cm² to around 100 cm², or around 3 cm² to around 50 cm². For example, as the substrate, a circular culture dish having a diameter of 10 cm can be mentioned. In this case, the area is 56.7 cm².

The substrate may be coated with serum. By using a substrate coated with serum, a sheet-shaped cell culture with a higher density can be formed. The expression "coated with serum" means a state that a serum component adheres onto a surface of the substrate. Such a state can be obtained, for example, by processing a substrate with serum, without any limitation. The processing with serum includes bringing serum into contact with a substrate, and performing the incubation for a predetermined period of time as needed.

As the serum, heterologous serum and/or homologous serum can be used. In a case where a sheet-shaped cell culture is used for transplantation, the heterologous serum means serum derived from an organism of a species different from that of the recipient. For example, in a case where the recipient is a human, serum derived from a bovine or a horse, for example, fetal bovine serum/fetal calf serum (FBS/FCS), calf serum (CS), horse serum (HS), or the like corresponds to the heterologous serum. Further, the expression "homologous serum" means serum derived from an organism of the same species as that of the recipient. For example, in a case where the recipient is a human, human serum corresponds to the homologous serum. The homologous serum includes self serum (also referred to as "autologous serum"), that is, serum derived from the recipient, and homologous non-autologous serum derived from an individual of the same species other than the recipient. Note that in the present specification, serum other than self serum, that is, heterologous serum and homologous non-autologous serum may be collectively referred to as "non-self serum".

The serum for coating on a substrate is commercially available, or can be prepared from the blood collected from a desired organism by a conventional method. Specifically, for example, a method in which the collected blood is left to stand at room temperature for around 20 minutes to around 60 minutes so as to be coagulated, the coagulated blood is centrifuged at around 1000×g to around 1200×g, and a supernatant is collected, or the like can be mentioned.

In a case where the incubation is performed on a substrate, serum may be used in undiluted form, or may be diluted for use. The serum can be diluted, without any limitation, in any medium, for example, water, a saline solution, various buffer solutions (for example, PBS, HBSS and the like), various liquid media (for example, DMEM, MEM, F12, DMEM/F12, DME, RPMI1640, MCDB (such as MCDB102, 104, 107, 120, 131, 153, or 199), L15, SkBM, or RITC80-7) or the like. The dilution concentration is not particularly limited as long as the serum component can adhere onto a substrate, and is, for example, around 0.5% to around 100% (v/v), preferably around 1% to around 60% (v/v), and more preferably around 5% to around 40% (v/v).

The incubation time is also not particularly limited as long as the serum component can adhere onto a substrate, and is, for example, around 1 hour to around 72 hours, preferably around 2 hours to around 48 hours, more preferably around 2 hours to around for 24 hours, and furthermore preferably around 2 hours to around 12 hours. The incubation temperature is also not particularly limited as long as the serum component can adhere onto a substrate, and is, for example, around 0°C to around 60°C, preferably around 4°C to around 45°C, and more preferably room temperature to around 40°C.

The serum may be discarded after incubation. As the technique for discarding the serum, suction with a pipette or the like, or a conventionally-used technique for discarding liquid such as decantation can be used. In a preferred embodiment of the present disclosure, after the serum is discarded, the substrate may be washed with a serum-free washing solution. The serum-free washing solution is not particularly limited as long as it is a liquid medium that does not contain serum and does not adversely affect the serum component adhered onto a substrate, and the washing can be performed, without any limitation, for example, by water, a saline solution, various buffer solutions (for example, PBS, HBSS and the like), various liquid media (for example, DMEM, MEM, F12, DMEM/F12, DME, RPMI1640, MCDB (such as MCDB102, 104, 107, 120, 131, 153, or 199), L15, SkBM, or RITC80-7), or the like. As the washing technique, without any limitation, a conventionally-used technique for washing a substrate, for example, a technique in which a serum-free washing solution is added onto a substrate, stirred for a predetermined time (for example, around 5 seconds to around 60 seconds), and then discarded, or the like can be used.

In the present disclosure, the substrate may be coated with a growth factor. In this regard, the expression "growth factor" means any substance that promotes proliferation of cells as compared with a substrate that is not coated with the growth factor, and examples of the growth factor include epidermal growth factor (EGF), vascular endothelial growth factor (VEGF), and fibroblast growth factor (FGF). The technique for coating on a substrate with a growth factor, the discarding technique, and the washing technique are basically the same as those of serum except that the dilution concentration at the time of incubation is, for example, around 0.000 1 µg/mL to around 1 µg/mL, preferably around 0.0005 µg/mL to around 0.05 µg/mL, and more preferably around 0.001 µg/mL to around 0.01 µg/mL.

In the present disclosure, the substrate may be coated with a steroid drug. In this regard, the expression "steroid drug" refers to a compound that does not exert an adverse effect on the living body, such as adrenocortical insufficiency, or Cushing syndrome, among the compounds having a steroid nucleus. Examples of the compound include, but are not limited to, cortisol, prednisolone, triamcinolone, dexamethasone, and betamethasone. The technique for coating on a substrate with a steroid drug, the discarding technique, and the washing technique are basically the same as those of serum except that the dilution concentration at the time of incubation is, for example, 0.1 µg/mL to around 100 µg/mL, preferably around 0.4 µg/mL to around 40 µg/mL, and more preferably around 1 µg/mL to around 10 µg/mL, as dexamethasone.

The substrate may be coated with any one of the serum, the growth factor, and the steroid drug, or may be coated with any combination of the serum, the growth factor, and the steroid drug, that is, a combination of the serum and the growth factor, a combination of the serum and the steroid drug, a combination of the serum, the growth factor, and the steroid drug, or a combination of the growth factor and the steroid drug. In a case of coating with multiple components, the coating with these components may be performed at the same time by mixing the components with each other, or may be performed in separate steps.

The substrate may be seeded with cells immediately after being coated with serum and the like, or may also be stored after being coated and then seeded with cells. The coated substrate can be stored for a long period of time, for example, by keeping the substrate at around 4°C or less, preferably around -20°C or less, and more preferably around -80°C or less.

In one embodiment, the present invention is a cell culture for treating inflammatory disease generated in the lower gastrointestinal tract. In the present invention, the lower gastrointestinal tract refers to the small intestine and the large intestine. In this regard, the small intestine includes the jejunum and the ileum, and the large intestine includes the appendix, the cecum, the ascending colon, the transverse colon, the descending colon, the sigmoid colon, the upper S rectum, the upper rectum, the lower rectum, and the anal canal. Examples of the inflammatory disease generated in the lower part of the gastrointestinal tract include, but are not limited to, inflammation due to a malignant tumor such as small intestine cancer, or colon cancer, infectious enteritis, diverticulitis, and inflammatory bowel disease.

In one embodiment, the present invention is a cell culture for treating inflammatory bowel disease. In the present invention, the inflammatory bowel disease refers to chronic or ameliorated/relapsed inflammatory disease in the intestinal tract, and specifically refers to ulcerative colitis, Crohn disease, or intestinal Behcet disease.

In one embodiment, the cell culture according to the present invention is transplanted to the serosal side of the gastrointestinal tract, that is, the outside of the gastrointestinal tract. The cell culture may be transplanted away from the inflamed site as long as the effect of the present invention is exerted, but it is preferable that all or part of the cell culture is transplanted to a position opposite to the position corresponding to the inflamed site across the muscle layer. In one embodiment, the cell culture may be transplanted to the serosal side of the gastrointestinal tract except for the position corresponding to the inflamed site. In one embodiment, the cell culture contains an extracellular matrix. In one embodiment of the present invention, the cell culture of the present invention is a sheet-shaped cell culture.

In a case where there are multiple inflamed sites, one cell culture may be transplanted to the multiple inflamed sites, or multiple cell cultures depending on the number of inflamed sites may be transplanted to the multiple inflamed sites, respectively. In a case where multiple cell cultures are transplanted, the multiple cell cultures may be each independently transplanted away from the inflamed site as long as the effect of the present invention is exerted, but it is preferable that all or part of the multiple cell cultures are transplanted to a position opposite to the position corresponding to the inflamed site across the muscle layer.

In order to transplant the cell culture according to the present invention to a subject, the morphology is not particularly limited, but is preferably morphology of a sheet-shaped cell culture.

The cell culture according to the present invention secretes myokine. The cell culture according to the present invention secretes myokine even in the morphology of a sheet-shaped cell culture. Further, by transplanting a sheet-shaped cell culture that secretes myokine to a subject having inflammatory disease, the inflammatory disease can be treated.

Without being bound by any particular theory, the action of the cell culture according to the present invention on inflammatory disease is considered because myokine secreted from the cell culture according to the present invention acts directly and/or indirectly on the cells at an inflamed site, and the proliferative effect of intestinal epithelial cells, the anti-inflammatory effect, and the anti-apoptotic effect are exerted. Further, in a case where the cell culture according to the present invention contains an extracellular matrix, it is considered that the bioadhesiveness of the cell culture to a transplant site is enhanced, and when myokine secreted from the cell culture is more produced, the myokine acts efficiently on the cells at an inflamed site.

Another aspect of the present invention relates to a method for producing a sheet-shaped cell culture containing cells derived from skeletal muscle, for treating inflammatory disease, including seeding cells on a substrate, forming the seeded cells into a sheet-shaped cell culture, and detaching the formed sheet-shaped cell culture from the substrate.

The sheet-shaped cell culture can be produced by any known method including seeding cells on a substrate, forming the seeded cells into a sheet-shaped cell culture, and detaching the formed sheet-shaped cell culture from the substrate.

As described above, in the present invention, the sheet-shaped cell culture can be produced by any method known to a person skilled in the art (see, for example, Patent Literature 1, JP 2010-081829 A, JP 2011-110368 A, and the like). The method for producing a sheet-shaped cell culture typically includes, but is not limited to, a step of seeding cells on a substrate, a step of forming the seeded cells into a sheet-shaped cell culture, and a step of detaching the formed sheet-shaped cell culture from the substrate.

Seeding of cells on a substrate can be performed by any known technique under any known conditions. The seeding of cells on a substrate may be performed, for example, by injecting a cell suspension in which cells are suspended in a liquid culture medium into a substrate (culture container). For the injection of the cell suspension, an instrument suitable for the injection operation of the cell suspension, such as a dropper or a pipette, can be used.

In one embodiment, the seeding can be performed at a density of around 7.1 × 10⁵ cells/cm² to around 3.0 × 10⁶ cells/cm², around 7.3 × 10⁵ cells/cm² to around 2.8 × 10⁶ cells/cm², around 7.5 × 10⁵ cells/cm² to around 2.5 × 10⁶ cells/cm², around 7.8 × 10⁵ cells/cm² to around 2.3 × 10⁶ cells/cm², around 8.0 × 10⁵ cells/cm² to around 2.0 × 10⁶ cells/cm², around 8.5 × 10⁵ cells/cm² to around 1.8 × 10⁶ cells/cm², around 9.0 × 10⁵ cells/cm² to around 1.6 × 10⁶ cells/cm², around 1.0 × 10⁶ cells/cm² to around 1.6 × 10⁶ cells/cm², or the like.

Further, another aspect of the present invention relates to a kit for producing a cell culture containing cells derived from skeletal muscle, or the like, for treating the above-described inflammatory disease, including cells for producing a cell culture, and a culture medium and a substrate for culturing the cells.

The kit according to the present invention may further includes, but is not limited to, for example, a washing solution, a buffer solution, a tube, an instrument used for cell culture, a transport container, instructions on the use method, and the like.

Examples of the instrument used for cell culture include a pipette, a cell strainer, and a cell scraper. Examples of the instructions on the use method include instructions for use, and a medium on which information about the production method and the use method has been recorded, for example, a flexible disk, a CD, a DVD, a Bluray disc, a memory card, a USB flash drive, or the like.

Further, another aspect of the present invention relates to a method for treating inflammatory disease, including performing transplantation of a cell culture containing cells derived from skeletal muscle. In one embodiment, the cell culture according to the present invention can be transplanted to or around the inflamed site to suppress inflammation in inflammatory disease. The cells forming the cell culture according to the present invention contain cells derived from skeletal muscle by 50% or more, 60% or more, 65% or more, 70% or more, 75% or more, 80% or more, 85% or more, or 90% or more, and preferably 60% or more. In one embodiment of the present invention, the cell culture is a sheet-shaped cell culture.

In one embodiment, the treatment of inflammatory disease is treatment of inflammatory disease generated in the lower gastrointestinal tract. In one embodiment, the treatment of inflammatory disease is treatment of inflammatory bowel disease.

In one embodiment, the cell culture according to the present invention is transplanted to the serosal side, that is, the outside of the gastrointestinal tract. The cell culture may be transplanted away from the inflamed site as long as the effect of the present invention is exerted, but it is preferable that all or part of the cell culture is transplanted to a position opposite to the position corresponding to the inflamed site across the muscle layer. In one embodiment, the cell culture may be transplanted to the serosal side of the gastrointestinal tract except for the position corresponding to the inflamed site. In one embodiment, the cell culture contains an extracellular matrix. In one embodiment of the present invention, the cell culture of the present invention is a sheet-shaped cell culture.

In a case where there are multiple inflamed sites, one cell culture may be transplanted to the multiple inflamed sites, or multiple cell cultures depending on the number of inflamed sites may be transplanted to the multiple inflamed sites, respectively. In a case where multiple cell cultures are transplanted, the multiple cell cultures may be each independently transplanted away from the inflamed site as long as the effect of the present invention is exerted, but it is preferable that all or part of the multiple cell cultures are transplanted to a position opposite to the position corresponding to the inflamed site across the muscle layer.

In one embodiment, in a case where the cell culture according to the present invention is a sheet-shaped cell culture, the sheet-shaped cell culture may have a support layer with a biodegradable gel such as a fibrin gel at the time of transplantation. As the method for forming the support layer, for example, although the method is not limited to, a method for forming a fibrin gel layer by adding a fibrinogen solution dropwise onto a sheet-shaped cell culture, and then spraying a thrombin solution (see, JP 6495603 B), or the like can be mentioned.

### Examples

### Example 1: Analysis of culture supernatant of sheet-shaped cell culture containing human myoblast cells

### (1) Preparation of sheet-shaped cell culture

The cryopreserved human myoblast cells (derived from a human skeletal muscle sample) were thawed at 37°C, and washed twice with a buffer solution containing 0.5% serum albumin. 5 × 10⁶ to 5 × 10⁷ cells were suspended in a 20% serum-containing medium, seeded in a flask, and then cultured for 2 to 3 days.

Into UpCell (registered trademark) (3.5-cm dish or 24-multiwell, CellSeed Inc.), a 20% serum-containing medium was added so as to cover the entire culture surface, and the processing was performed in an environment of 37°C and 5% CO₂ for 3 hours to 3 days. After the processing, the added medium was discarded.

The cultured cells were collected, suspended in a 20% serum-containing medium, and seeded in processed UpCell (registered trademark) at a density of 2 to 20 × 10⁵ cells/cm², and then the sheet-forming incubation was performed for around 1 day in an environment of 37°C and 5% CO₂.

### (2) Assay of secreted cytokine

By using Bio-Plex (trademark) Assay Kits (manufactured by Bio-Rad Laboratories, Inc.), the culture supernatant of the sheet-shaped cell culture prepared in (1) was analyzed for 27 kinds of cytokines in accordance with the instructions of manufacturer.

### (3) Results

The results are shown in Fig. 1. In the culture supernatant of the sheet-shaped cell culture containing human myoblast cells, VEGF, IL-6, IL-8, RANTES, G-CSF, IL-12, IL-10, IP-10, and the like were secreted.

### Example 2: Transplant test in mouse model of colitis

### (1) Preparation of sheet-shaped cell culture

Skeletal muscle was collected from the lower limb of an 18-week old C57BL/6 mouse (CHARLES RIVER LABORATORIES JAPAN, INC.), processed with a solution containing collagenase and trypsin, and dispersed to single cells. Such cells were cultured in a 20% FBS-containing MCDB131 medium under the conditions of 37°C and 5% CO₂ until the confluent was obtained, and the cells were collected. 1 × 10⁶ cells of the collected cells were seeded in a 48-well temperature-responsive culture container (UpCell (registered trademark)), and cultured in a 20% FBS-containing DMEM/F12 medium for 6 hours or more, and the sheet-forming incubation was performed. After that, by lowering the temperature to 20°C, a sheet-shaped cell culture was detached from the temperature-responsive culture container, and collected.

### (2) Test method

The test was carried out in accordance with the following method. The group composition of mice and the plan of autopsy and transplantation time are shown in Fig. 2.

In order to induce colitis, a 2% dextran sulfate sodium (DSS, MP Bio Japan K.K.) was orally administered to 7-week old C57BL/6 mice (Charles River Laboratories Japan, Inc.) in free drinking water for 4 days. The mice were randomly divided into a follow-up group and a test group. In the follow-up group, the autopsy was performed to examine the condition of the tissue on the 4th day, 10th day, 17th day, and 31st day counted from the start date of the free drinking water (water-free drinking) (hereinafter, the number of days is counted from the start date of the free drinking water). The test group was further divided into a sheet-shaped cell culture transplantation group and a sham surgery group. The mice in the sheet-shaped cell culture transplantation group were subjected to laparotomy under general anesthesia on the 11th day, and the sheet-shaped cell culture prepared in (1) was transplanted to the serosal side of the gastrointestinal tract where the large intestine was inflamed. The mice in the sham surgery group were subjected to only laparotomy under general anesthesia without transplanting any sheet-shaped cell culture. An autopsy was performed on the mice in the sheet-shaped cell culture transplantation group and in the sham surgery group on the 31st day to examine the condition of the tissue. Further, the lower gastrointestinal tract was collected from the mice in both of the groups, and the length of the large intestine was measured. In addition, the colon tissue was collected from the lower gastrointestinal tract and stained with hematoxylin-eosin stain, and a stained tissue image was obtained.

Further, in addition to the above-described histological experiment, for the mice in the sheet-shaped cell culture transplantation group and in the sham surgery group, the changes in the body weight, the property of stool, and the degree of bloody stool were observed, and the inflammation score (DAI) was calculated in accordance with the following Table 1 on the 3rd day, 9th day, 16th day, and 23rd day after the start of the test. The total score is 12 points, and it is indicated that the higher the numerical value is, the more severe the degree of inflammation is.

### [Table 1]

**Table 1. Inflammation scores**

| Score | Weight decrease | Stool property | Bloody stool |
|---|---|---|---|
| 0 | less than 1% | Normal | Normal |
| 1 | 1 to 5% | | |
| 2 | 5 to 10% | Loose stool | Small amount |
| 3 | 10 to 20% | | |
| 4 | 20% or more | Watery stool | Large amount |

### (3) Results

A photograph of the lower gastrointestinal tract collected from a mouse in the sheet-shaped cell culture transplantation group is shown in (A) of Fig. 3, a photograph of the inner membrane of the gastrointestinal tract confirmed by cutting open the lower gastrointestinal tract is shown in (B) of Fig. 3, a photograph of the lower gastrointestinal tract taken out from a mouse in the sham surgery group is shown in (C) of Fig. 3, and a photograph of the inner membrane of the gastrointestinal tract confirmed by cutting open the lower gastrointestinal tract is shown in (D) of Fig. 3. The length of the large intestine of a mouse in the sheet-shaped cell culture transplantation group was 73 mm, and the length of the large intestine of a mouse in the sham surgery group was 67 ± 5 mm.

Among the stained tissue images obtained from the mice in the sheet-shaped cell culture transplantation group and in the sham surgery group, on which an autopsy was performed on the 31st day, a stained tissue image obtained from a mouse in the sheet-shaped cell culture transplantation group is shown in Fig. 4A, and an enlarged image of the dashed line portion is shown in Fig. 4B. Further, a stained tissue image obtained from a mouse in the sham surgery group is shown in Fig. 4C, and an enlarged image of the dashed line portion is shown in Fig. 4D.

The histopathological evaluation of the tissues of the mice in the follow-up group, on which an autopsy was performed on the 4th day, 10th day, 17th day, and 31st day, and the mice in the sheet-shaped cell culture transplantation group and in the sham transplantation group, on which an autopsy was performed on the 31st day, is shown in the following Table 2.

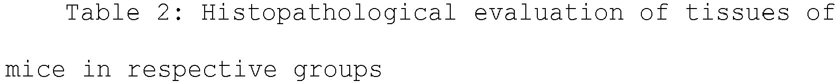

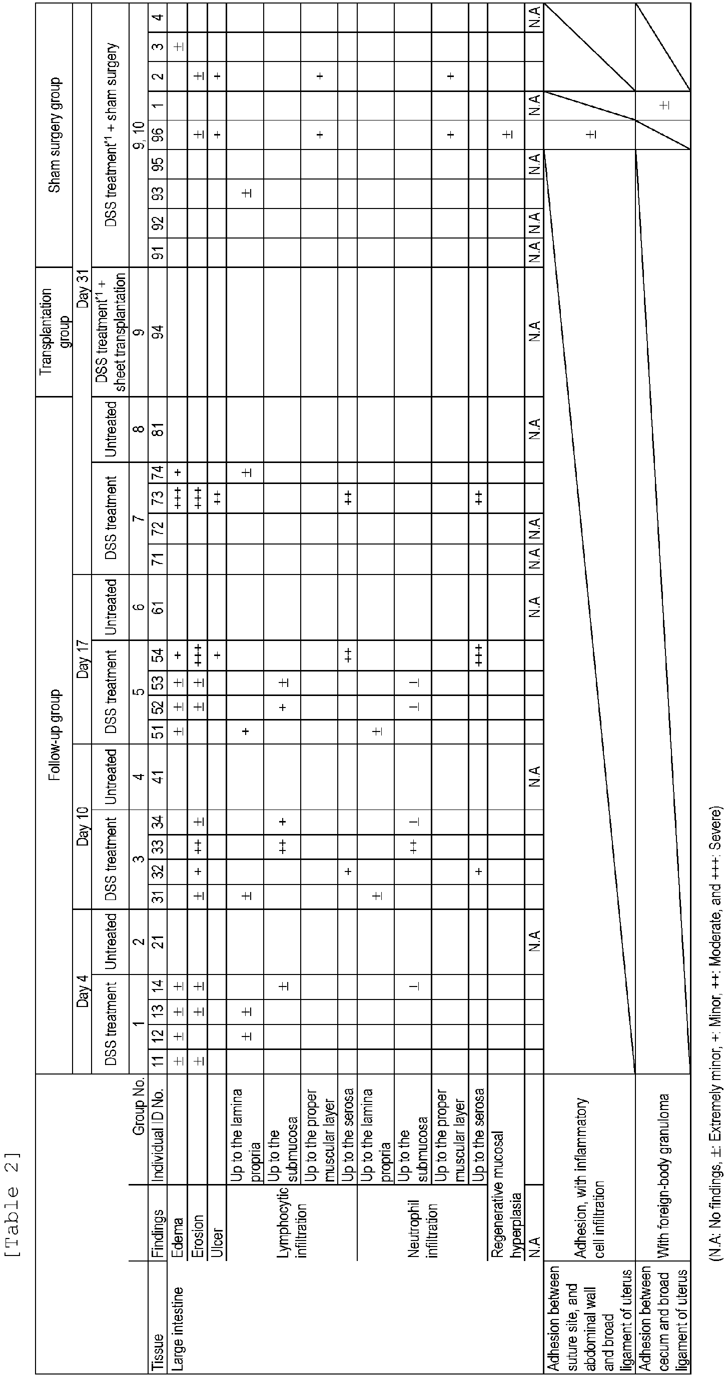

Erosion, ulcer, lymphocytic infiltration, and neutrophil infiltration were confirmed in the large intestine of the mice in the sham transplantation group on which an autopsy was performed on the 31st day, but abnormalities were not confirmed in the large intestine of the mouse in the sheet-shaped cell culture transplantation group on which an autopsy was performed on the 31st day.

The changes in the body weight of the mice in the sheet-shaped cell culture group and in the sham surgery group are shown in Fig. 5. Further, the changes in the body weight of the mice in the sheet-shaped cell culture transplantation group and in the sham surgery group from the 11th day after the start of the test based on the body weight of each mouse on the 11 days after the start of the test are shown in Fig. 6. The squares indicate the mouse in the sham surgery group, and the black circles indicate the mouse in the sheet-shaped cell culture transplantation group. After the transplantation of a sheet-shaped cell culture, the mouse in the sheet-shaped cell culture transplantation group showed a significant increase in the body weight as compared with the mouse in the sham surgery group.

The inflammation scores (DAI) for the sheet-shaped cell culture transplantation group and the sham surgery group are shown in Fig. 7. The squares indicate the sham surgery group, and the black circles indicate the sheet-shaped cell culture transplantation group. In the sham surgery group, the scores were high also on the 16th day and 23rd day after the laparotomy. On the other hand, in the sheet-shaped cell culture transplantation group, surprisingly, the score on the 16th day after the laparotomy was 0, and the score on the 23rd day was also 0.

## Claims

1. A cell culture for treating inflammatory disease, comprising cells derived from skeletal muscle.

2. The cell culture according to claim 1, wherein the cell culture is a sheet-shaped cell culture.

3. The cell culture according to claim 1 or 2, wherein the cell culture secretes myokine.

4. The cell culture according to any one of claims 1 to 3, wherein the inflammatory disease is generated in the lower gastrointestinal tract.

5. The cell culture according to any one of claims 1 to 4, wherein the inflammatory disease is inflammatory bowel disease.

6. The cell culture according to any one of claims 1 to 5, wherein the cell culture is used for transplantation to the serosal side of the gastrointestinal tract.

7. A method for producing the sheet-shaped cell culture according to any one of claims 2 to 6, comprising:
seeding cells on a substrate;
forming the seeded cells into a sheet-shaped cell culture; and
detaching the formed sheet-shaped cell culture from the substrate.

8. A kit for producing the cell culture according to any one of claims 1 to 6, comprising: cells; and a culture medium and a substrate, for culturing the cells.

9. A method for treating inflammatory disease, comprising transplanting a cell culture containing cells derived from skeletal muscle.

10. The method according to claim 9, wherein the cell culture is a sheet-shaped cell culture.

11. The method according to claim 9 or 10, wherein the cell culture secretes myokine.

12. The method according to any one of claims 9 to 11, wherein the inflammatory disease is generated in the lower gastrointestinal tract.

13. The method according to any one of claims 9 to 12, wherein the inflammatory disease is inflammatory bowel disease.

14. The method according to any one of claims 9 to 13, wherein transplantation of the cell culture is transplantation to the serosal side of the gastrointestinal tract.
